# EUROPÄISCHE PATENTSCHRIFT

(11) **EP 2 195 284 B1**
(45) Veröffentlichungstag und Bekanntmachung des Hinweises auf die Patenterteilung: **22.03.2017**
(21) Anmeldenummer: 08802162.1
(22) Anmeldetag: 13.09.2008
(51) Int. Cl.: C07C 227/16, C07C 229/30

(54) **VERFAHREN ZUR HERSTELLUNG VON 2-DIHALOGENACYL-3-AMINO-ACRYLSÄURE-DERIVATEN**
PROCESS FOR PREPARING 2-DIHALOACYL-3-AMINOACRYLIC ACID DERIVATIVES
PROCÉDÉ DE PRÉPARATION DE DÉRIVÉS D'ACIDE 2-DIHALOGÈNE-ACYL-3-AMINO-ACRYLIQUE

(30) Priorität: 26.09.2007 EP 07117232
(43) Veröffentlichungstag der Anmeldung: 16.06.2010
(73) Patentinhaber: Bayer Intellectual Property GmbH, 40789 Monheim am Rhein (DE)
(72) Erfinder: PAZENOK, Sergii, 42699 Solingen (DE); LUI, Norbert, 51519 Odenthal (DE)
(74) Vertreter: BIP Patents
(86) Internationale Anmeldenummer: PCT/EP2008/007612
(87) Internationale Veröffentlichungsnummer: WO 2009/043444

(56) Entgegenhaltungen:
- WO-A-2005/042468

## Beschreibung

Die vorliegende Erfindung betrifft ein Verfahren zur Herstellung von Hydrochlorid-freien 2-Dihalogenacyl-3-amino-acrylsäureestern durch Umsetzung von Säurefluoriden mit Dialkylaminoacrylsäure-Derivaten.

2-Dihalogenacyl-3-amino-acrylsäureester der Formel (I) sind wertvolle Zwischenprodukte für die Herstellung von Dihalogenmethyl-substituierten Pyrazolylcarbonsäure-Derivaten, welche als Vorstufen von fungiziden Wirkstoffen dienen können (vgl. WO 03/070705).

Aus Tetrahedron Lett. 1996, 37, 8751-8754 ist bereits bekannt, dass man trihalogenacylierte Aminoacrylsäureester erhält, wenn man die entsprechenden Chloracroleine mit einem substituierten Amin umsetzt. Die als Ausgangsstoffe benötigten Chloracroleine werden dabei aus den entsprechenden Trihalogenacetoacetaten mittels Vilsmeier-Reaktion gewonnen. Dieses Verfahren hat zum einen den Nachteil, dass bei der Vilsmeier-Reaktion Phosphoroxidtrichlorid eingesetzt werden muss, und zum anderen die Gesamtausbeuten großtechnisch nicht zufriedenstellend sind.

EP-A-1 000 926 lehrt, dass man Trihaloacylaminopropenoate erhält, indem man Trihalogenacetoacetate mit Dialkylformamidacetalen umsetzt. Nachteilig ist hier, dass als Nebenprodukt die deacylierte Verbindung auftritt und vom gewünschten Produkt abgetrennt werden muss, was zu zusätzlichen Kosten und Ausbeuteverlusten führt.

WO 03/051820 lehrt, dass man 2-Perhalogenacyl-3-aminoacrylsäure-Derivate erhalten kann, indem man 3-Aminoacrylsäureester mit Perhalogenalkylcarbonsäureanhydriden umsetzt. Das beschriebene Verfahren eignet sich jedoch nicht zum Herstellen von Dihalogenacyl-substituierten Aminoacrylsäure-Derivaten, da in Anwesenheit eines α-Wasserstoffs, in Gegenwart von Triethylamin, Chlorwasserstoff abgespalten wird. Die so entstehenden Dihalogenketene sind sehr instabile Verbindungen (vgl. J. Org. Chem. 1968, 33, 816), welche zur Polymerisation neigen.

Aus WO 2005/042468 ist bekannt, dass man 2-Perhalogenacyl-3-aminoacrylsäure-Derivate erhalten kann, indem man 3-Aminoacrylsäureester mit Säurehalogeniden in Gegenwart einer organischen Base umsetzt. Bei diesen Verfahren entstehen in equimolaren Mengen Salze, z. B. Hydrochloride, die vom dem Produkt durch Filtration oder wässrige Aufarbeitung abgetrennt werden müssen. Wird in Folge, im Zusammenhang mit der Erfindung, der Begriff "Hydrochloride" verwendet, so sollen von dem Begriff alle durch die Reaktion mit der Base entstehenden Verunreinigungen, z.B. Hydrochloride, HCl, sonstige Salze, umfasst sein. Allein durch Filtration kann eine vollständige Abtrennung der Produkte von den Hydrochloriden jedoch nicht erfolgen, so dass gewisse Mengen der Hydrochloride im Produkt verbleiben. Auch eine wässrige Aufarbeitung ist in vielen Fällen ungeeignet da zahlreiche 2-Perhalogenacyl-3-amino-acrylsäuren, wie zum Beispiel 2,2-Difluoracetyl-3-amino-acrylsäure, hydrolyseempfindlich sind.

Außerdem verteuert die Anwendung einer organischen Base das Verfahren und führt zu zusätzlichen Abfällen.

Die vollständige Entfernung der Hydrochloride von den 2-Perhalogenacyl-3-aminoacrylsäureestern ist jedoch von großer synthetischer Bedeutung, da bei deren Umsetzung mit Alkylhydrazinen die Regioselektivität des Ringschlusses durch die Anwesenheit von HCl oder Hydrochloriden beeinträchtigt wird. So wurde beobachtet, dass bei Anwesenheit auch nur geringer Mengen Hydrochloride, der Anteil des unerwünschten regioisomeren 5-Haloalkyl-4-carbonsäurepyrazol überproportional wächst.

So lehrt beispielsweise die US 5,498,624, dass 3-Difluormethyl-pyrazol-Derivate erhalten werden können, wenn man 2-(Difluoracetyl)-3-alkoxyacrylate mit Hydrazinen in protischen Lösungsmitteln umsetzt. Auch hier lassen die Ausbeuten des Verfahrens zu wünschen übrig, da zu einem hohen Prozentsatz die unerwünschten isomeren Pyrazole entstehen und bei der Isolierung des gewünschten Isomeren weitere Verluste entstehen. Die technische Anwendung eines solchen Verfahrens ist daher aus ökonomischen Gründen kaum möglich.

Bei der Ringschluss-Reaktion von Alkoxyacrylaten mit Hydrazinderivaten entsteht zu einem hohen Prozentsatz (bis 88 %) das nicht erwünschte 5-Haloalkyl-4-carbonsäurepyrazol (vgl. J. Het. Chem. 1987, 24, 693).

Die Dihalogenmethylalkoxyacrylate werden aus Dihalogenacetessigester hergestellt. Dihalogenacetessigester sind kommerziell nicht verfügbar und deren Herstellung ist technisch Anspruchsvoll, da sie beispielsweise den Einsatz von Keten erfordert. Die Verbindungen sind daher nicht in wirtschaftlicher Weise herstellbar.

Aus WO 03/051820 ist bekannt, dass man 2-Perhalogenacyl-3-aminoacrylsäure-Derivate mit Hydrazinen zu 3-Perhalogen-substituierten Pyrazolen umsetzen kann. Durch Verwendung eines aprotischen Lösungsmittels kann zwar die Bildung des unerwünschten Isomeren herabgesetzt werden, diese ist aber bei Anwendung auf die erfindungsgemäßen Dihalogen-Verbindungen immer noch beträchtlich.

Der vorliegenden Erfindung lag daher die Aufgabe zugrunde, neue und wirtschaftlichere Verfahren zur Verfügung zu stellen, durch die 2-Dihalogenacyl-3-aminoacrylsäureester, die frei von den zuvor beschriebenen Hydrochlorid-Verunreinigungen sind, mit hoher Gesamtausbeute erhalten werden können.

Die Aufgabe wurde gelöst durch ein Verfahren zum Herstellen von 2-Dihalogenacyl-3-aminoacrylsäure-Derivaten der Formel (I) 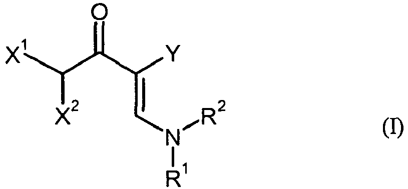 in welcher
- R¹ und R²: unabhängig voneinander ausgewählt sind aus C₁₋₁₂-Alkylresten, C₅₋₁₈-Aryl-, C₇₋₁₉-Alkylaryl- oder C₇₋₁₉-Arylalkyresten; oder
- R¹ und R²: zusammen mit dem Stickstoffatom, an das sie gebunden sind, einen 5- bis 6-gliedrigen Ring bilden können, der gegebenenfalls ein oder zwei weitere Hetero-Atome, ausgewählt aus O, S und einer SO₂-Gruppe enthalten kann;
- Y: ausgewählt ist aus (C=O)OR³, CN und (C=O)NR⁴R⁵, wobei R³, R⁴ und R⁵ unabhängig von einander ausgewählt sind aus C₁₋₁₂-Alkylresten, C₅₋₁₈-Aryl-, C₇₋₁₉-Alkylaryl- oder C₇₋₁₉-Arylalkyresten und R⁴ und R⁵ gemeinsam mit dem N-Atom, an das sie gebunden sind und/oder weiteren Atomen, die ausgewählt sind aus C, N, O und S einen fünf- oder sechsgliedrigen Ring bilden können, der mit C₁₋₆-Alkylresten substituiert sein kann;
- X¹ und X²: unabhängig voneinander für Fluor, Chlor, Brom oder Iod stehen,
durch Umsetzung von Säurefluoriden der Formel (II) 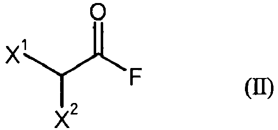 in welcher X¹ und X² die oben genannten Bedeutungen haben, mit 3-Amino-acrylsäure-Derivaten der Formel (III) 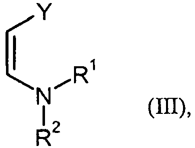 in welcher R¹, R² und Y die oben genannte Bedeutungen haben, dadurch gekennzeichnet, dass die Umsetzung in Abwesenheit von Basen erfolgt.

### Allgemeinen Definitionen

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen sind beispielsweise ausgewählt aus Trifluormethyl (CF₃), Difluormethyl (CHF₂), CF₃CH₂, ClCH₂, CF₃CCl₂, CF₃CHF.

Alkyl-Gruppen sind im Zusammenhang mit der vorliegenden Erfindung, soweit nicht abweichend definiert, lineare, verzweigte oder ringförmige gesättigte Kohlenwasserstoff-Gruppen.

Die Definition C₁-C₁₂-Alkyl umfasst den größten hierin definierten Bereich für einen AlkylGruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Alkenyl-Gruppen sind im Zusammenhang mit der vorliegenden Erfindung, soweit nicht abweichend definiert, lineare, verzweigte oder ringförmige Kohlenwasserstoff-Gruppen, die wenigstens eine einfache Unsättigung (Doppelbindung) enthalten.

Die Definition C₂-C₁₂-Alkenyl umfasst den größten hierin definierten Bereich für einen AlkenylGruppe. Im Einzelnen umfasst diese Definition beispielsweise die Bedeutungen Vinyl; Allyl (2-Propenyl), Isopropenyl (1-Methylethenyl); But-1-enyl (Crotyl), But-2-enyl, But-3-enyl; Hex-1-enyl, Hex-2-enyl, Hex-3-enyl, Hex-4-enyl, Hex-5-enyl; Hept-1-enyl, Hept-2-enyl, Hept-3-enyl, Hept-4-enyl, Hept-5-enyl, Hept-6-enyl; Oct-1-enyl, Oct-2-enyl, Oct-3-enyl, Oct-4-enyl, Oct-5-enyl, Oct-6-enyl, Oct-7-enyl; Non-1-enyl, Non-2-enyl, Non-3-enyl, Non-4-enyl, Non-5-enyl, Non-6-enyl, Non-7-enyl, Non-8-enyl; Dec-1-enyl, Dec-2-enyl, Dec-3-enyl, Dec-4-enyl, Dec-5-enyl, Dec-6-enyl, Dec-7-enyl, Dec-8-enyl, Dec-9-enyl; Undec-1-enyl, Undec-2-enyl, Undec-3-enyl, Undec-4-enyl, Undec-5-enyl, Undec-6-enyl, Undec-7-enyl, Undec-8-enyl, Undec-9-enyl, Undec-10-enyl; Dodec-1-enyl, Dodec-2-enyl, Dodec-3-enyl, Dodec-4-enyl, Dodec-5-enyl, Dodec-6-enyl, Dodec-7-enyl, Dodec-8-enyl, Dodec-9-enyl, Dodec-10-enyl, Dodec-11-enyl; Buta-1,3-dienyl, Penta-1,3-dienyl.

Alkinyl-Gruppen sind im Zusammenhang mit der vorliegenden Erfindung, soweit nicht abweichend definiert, lineare, verzweigte oder ringförmige Kohlenwasserstoff-Gruppen, die wenigstens eine zweifache Unsättigung (Dreifachbindung) enthalten.

Die Definition C₂-C₁₂-AMnyl umfasst den größten hierin definierten Bereich für einen Alkinyl-Gruppe. Im Einzelnen umfasst diese Definition beispielsweise die Bedeutungen Ethinyl (Acetylenyl); Prop-1-inyl und Prop-2-inyl.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S aufweisen können.

Die Definition C₅₋₁₈-Aryl umfasst den größten hierin definierten Bereich für einen Aryl-Gruppe mit 5 bis 18 Atomen. Im Einzelnen umfasst diese Definition beispielsweise die Bedeutungen Cyclopentadienyl, Phenyl, Cycloheptatrienyl, Cyclooctatetraenyl, Naphthyl und Anthracenyl.

Arylalkyl-Gruppen (Aralkyl-Gruppen) sind im Zusammenhang mit der vorliegenden Erfindung, soweit nicht abweichend definiert, durch Aryl-Gruppen substituierte Alkyl-Gruppen, die eine C₁₋₈-Alkylenkette aufweisen können und im Arylgerüst ein oder mehrere Heteroatome, die ausgewählt sind aus O, N, P und S aufweisen können.

Die Definition C₇₋₁₉-Aralkyl-Gruppe umfasst den größten hierin definierten Bereich für einen Arylalkyl-Gruppe mit insgesamt 7 bis 19 Atomen in Gerüst und Alkylenkette. Im Einzelnen umfasst diese Definition beispielsweise die Bedeutungen Benzyl- und Phenylethyl.

Alkylaryl-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst ein oder mehrere Heteroatome, die ausgewählt sind aus O, N, P und S aufweisen können.

Die Definition C₇₋₁₉-Alkylaryl-Gruppe umfasst den größten hierin definierten Bereich für einen Alkylaryl-Gruppe mit insgesamt 7 bis 19 Atomen in Gerüst und Alkylenkette. Im Einzelnen umfasst diese Definition beispielsweise die Bedeutungen Tolyl, 2,3-, 2,4-, 2,5-, 2,6-, 3,4- oder 3,5-Dimethylphenyl.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

Es wurde festgestellt, dass nach dem erfindungsgemäßen Verfahren hergestellten 2-Dihalogenacyl-3-dialkylamino-acrylsäure-Derivate, die frei von Hydrochlorid Verunreinigungen sind, mit hohen Ausbeuten und Selektivitäten (Verhältnis 3-Dihalogenmethyl-2H-pyrazol-4-carbonsäure-Derivate << 3-Dihalogenmethyl-1H-pyrazol-4-carbonsäure-Derivate) umgesetzt werden können. Die Gesamtreaktion folgt dem allgemeinen Reaktionsschema 1.

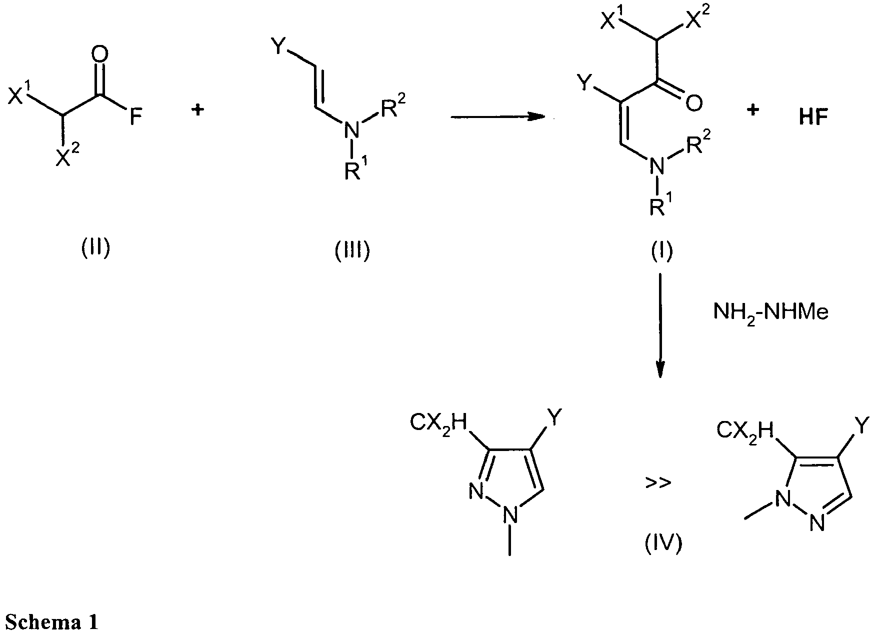

### Säurefluoride (II)

Die bei der Durchführung des erfindungsgemäßen Verfahrens als Ausgangsstoffe verwendeten Säurefluoride sind durch die Formel (II) allgemein definiert. In Formel (II) stehen die Reste X¹ und X² unabhängig voneinander für Fluor, Chlor, Brom und Jod vorzugsweise für Fluor, Chlor und Brom, besonders bevorzugt sind beide Gruppen Fluor.

Säurefluoride der Formel (II) sind bekannte Synthesechemikalien und können zum Beispiel auf einfache Weise aus Tetrafluorethylmethylethern, gemäß Schema III, hergestellt werden (D. England, J.Org.Chem. 1984, 49, 4007-4008).

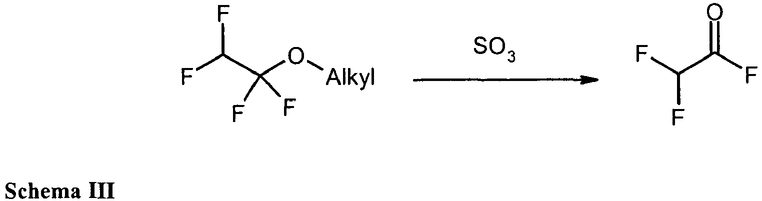

### Dialkylaminoacrylsäure-Derivate (III)

Die bei der Durchführung des erfindungsgemäßen Verfahrens als Ausgangsstoffe verwendeten Dialkylaminoacrylsäure-Derivate sind durch die Formel (III) allgemein definiert. In dieser Formel können
- R¹ und R²: unabhängig voneinander ausgewählt sein aus C₁₋₁₂-Alkylresten, C₅₋₁₈-Aryl-, C₇₋₁₉-Alkylaryl- oder C₇₋₁₉-Arylalkyresten, oder
- R¹ und R²: zusammen mit dem Stickstoffatom, an das sie gebunden sind, einen 5- bis 6-gliedrigen Ring bilden, der gegebenenfalls ein oder zwei weitere Hetero-Atome, ausgewählt aus O, S und einer SO₂-Gruppe enthalten kann,
- Y: ausgewählt sein aus Carbonsäureestergruppen ((C=O)OR³), Nitrilgruppen (CN) und Amidgruppen ((C=O)NR⁴R⁵), wobei R³, R⁴ und R⁵ unabhängig von einander ausgewählt sind aus C₁-₁₂Alkylresten, C₅₋₁₈-Aryl-, C₇₋₁₉-Alkylaryl- oder C₇₋₁₉-Arylalkylresten und R⁴ und R⁵ gemeinsam mit dem N-Atom, an das sie gebunden sind und/oder weiteren Atomen, die ausgewählt sind aus C, N, O und S einen fünf- oder sechsgliedrigen Ring bilden können, der mit C₁₋₆-Alkylresten substituiert sein kann.

Vorzugsweise können
- R¹ und R²: unabhängig voneinander ausgewählt sein aus Methyl, Ethyl, n-Propyl oder iso-Propyl,
- R¹ und R²: zusammen mit dem Stickstoffatom, an das sie gebunden sind, einen Piperidinyl- oder Pyrolidinyl-Ring bilden,
- Y: ausgewählt sein aus (C=O)OR³, wobei R³ ausgewählt ist aus Methyl, Ethyl, n-Propyl oder iso-Propyl.

Besonders bevorzugt können
- R¹ und R²: Methyl sein und
- Y: -(C=O)OC₂H₅ sein.

Dialkylaminoacrylsäureester der Formel (III) sind bekannte Synthesechemikalien und kommerziell erhältlich.

Beispiele für erfindungsgemäß geeignete Dialkylaminoacrylsäureester sind 3-(N,N-Dimethylamino)-acrylsäuremethylester, 3-(N,N-Dimethylamino)-acrylsäureethylester, 3-(N,N-Diethylamino)-acrylsäureethylester, 3-(N,N-Dimethylamino)-acrylsäurenitril, 3-(N,N-Dimethylamino)-acrylsäuredimethylamid und 3-(N,N-Dimethylamino)-acrylsäurediethylamid wobei 3-(N,N-Dimethylamino)-acrylsäureethylester besonders bevorzugt ist.

Verfahren zur Herstellung von Dialkylaminoacrylsäureestern sind im Stand der Technik, beispielsweise in EP-A-0 608 725, vorbeschrieben.

Verfahren zur Herstellung von Dialkylaminoacrylnitrilen sind im Stand der Technik, beispielsweise von Rene et al in Synthesis (1986), (5), 419-420 beschrieben.

Die Dialkylaminoacrylsäure-Derivate können, falls notwendig, beispielsweise destillativ gereinigt werden. Dies ist im Allgemeinen jedoch im Zusammenhang mit der erfindungsgemäßen Reaktion nicht erforderlich.

Das molare Verhältnis von Dialkylaminoacrylsäure-Derivate (III) zu eingesetzten Säurefluoriden (II) kann beispielsweise 0,5 bis 3, bevorzugt 0,8 bis 2, besonders bevorzugt 1,0 bis 1,5 betragen.

Das erfindungsgemäße Verfahren wird vorzugsweise in einem organischen Verdünnungs-/Lösungsmittels durchgeführt. Besonders eignen sich hierzu beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan. Besonders bevorzugt verwendet man Toluol, Xylol, Chlorbenzol, n-Hexan, Cyclohexan oder Methylcyclohexan, ganz besonders bevorzugt Toluol, Chlorbenzol, Acetonitril oder Xylol, Nitrile, Amide, Ether.

Das erfindungsgemäße Verfahren wird in Abwesenheit von Basen, d.h. ohne Zugabe von einer oder mehreren Basen durchgeführt. "Base" kann im Zusammenhang mit dem erfindungsgemäßen Verfahren jede anorganischen oder organischen Base sein.

Beispiele für organische Basen sind tertiäre Stickstoffbasen, wie z.B. tertiäre Amine, substituierte oder unsubstituierte Pyridine und substituierte oder unsubstituierte Chinoline, Triethylamin, Trimethylamin, Diisopropylethylamin, Tri-n-propylamin, Tri-n-butylamin, Tri-n-hexylamin, Tricyclohexylamin, N-Methyl-cyclohexylamin, N-Methylpyrrolidin, N-Methylpiperidin, N-Ethylpiperidin, N,N-Dimethylanilin, N-Methylmorpholin, Pyridin, 2-, 3-, 4-Picolin, 2-Methyl-5-ethyl-pyridin, 2,6-Lutidin, 2,4,6-Collidin, 4-Dimethylaminopyridin, Chinolin, Chinaldin, N,N,N,N-Tetramethylethyldiamin, N,N-Dimethyl-1,4-diazacyclohexan, N,N-Di-ethyl-1,4-diazacyclohexan, 1,8-Bis-(Dimethylamino)naphthalin, Diazabicyclooctan (DABCO), Diazabicyclononan (DBN) und Diazabicycloundecan (DBU).

Beispiele für anorganische Basen sind Alkali- oder Erdalkalimetallhydroxyde, Hydrogencarbonate oder Carbonate und sonstige anorganische wässrige Basen, bevorzugt sind z.B. Natriumhydroxid, Kaliumhydroxid, Lithiumhydroxid, Calciumhydroxid, Natriumcarbonat, Kaliumcarbonat, Natriumhydrogencarbonat, Kaliumhydrogencarbonat und Natriumacetat.

Um die Bildung von Salzen, z. B. von Hydrochloriden, zu vermeiden, wird das Verfahren in Abwesenheit von Basen durchgeführt. Dies bedeutet, dass besonders bevorzugt keine Basen in der Reaktionsmischung vorhanden sind. In der Praxis werden sich Spuren von Basen, die als Verunreinigungen anwesend sind, nicht vermeiden lassen. Basenfrei bedeutet daher, dass der Anteil von Basen in der Reaktionsmischung nicht größer als 1%, vorzugsweise nicht größer als 0,1 % besonders bevorzugt nicht größer als 0,01% bezogen auf die Reaktionsmischung ist.

Bei der Durchführung des erfindungsgemäßen Verfahrens muss innerhalb eines relativ geringen Temperaturbereichs gearbeitet werden. Im Allgemeinen arbeitet man bei Temperaturen von -50 bis 100°C, vorzugsweise -20°C bis +50°C, besonders bevorzugt bei Temperaturen von -10°C bis +45°C.

Das erfindungsgemäße Verfahren wird im Allgemeinen unter Normaldruck durchgeführt. Es ist jedoch auch möglich, unter erhöhtem Druck zu arbeiten, z. B. wenn das leicht flüchtige Difluoracetylfluorid eingesetzt wird. Erhöhter Druck bedeutet in diesem Zusammenhang 0,1 bis 5 bar, vorzugsweise 0,15 bis 4 bar, besonders bevorzugt 0,2 bis 1 bar.

Die Reaktionszeit ist nicht kritisch und kann in Abhängigkeit von der Ansatzgröße in einem größeren Bereich gewählt werden. Grundsätzlich liegt die Reaktonszeit im Bereich von 30 Minuten bis zu 4 h, vorzugsweise zwischen 45 min und 2 h.

Bei der Durchführung des erfindungsgemäßen Verfahrens (a) setzt man auf 1 Mol Säurefluorid der Formel (II) im Allgemeinen zwischen 0.5 Mol und 3 Mol, vorzugsweise zwischen 0.5 Mol und 1.5 Mol, besonders bevorzugt zwischen 0,9 Mol und 1,0 Mol eines Dialkylaminoacrylsäure-Derivats der Formel (III) ein.

Nach beendeter Reaktion kann das Reaktionsgemisch grundsätzlich ohne weitere Aufreinigung in die folgende Reaktionsstufe (Pyrazolsynthese) eingesetzt werden. Es ist als überraschend anzusehen das HF oder HF-Salze die Regioselktivität der Cyclisierung mit Alkylhydrazinen, insbesondere im Vergleich zu HCl oder den entsprechenden Hydrochloriden, nicht beeinträchtigen. Folglich kann das Verfahren ohne zwischenzeitliche Isolierung der 3-Dihalogenmethyl-1H-pyrazol-4-carbonsäure-Derivate der Formel (I) als Eintopf-Reaktion durchgeführt werden.

Selbst wenn die Reaktion in Gegenwart einer Base durchgeführt wird, ist die Entfernung von HF-Salzen ebenso nicht notwendig. Folglich kann die Umsetzung mit den Alkylhydrazinen, vorzugsweise mit Methylhydrazin, ebenfalls ohne Ausbeuteverluste durchgeführt werden.

### Herstellungsbeispiele

### Beispiel 1

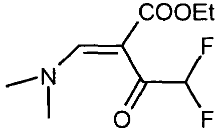

71.6 g (0.5 mol) Dimethylaminoacrylsäureethylester werden in 150 ml Toluol gelöst und die Mischung auf 0°C gekühlt. Anschließend werden innerhalb von 30-40 min bei 0-3°C 50 g (0.5 mol) Difluoracetylfluorid unter Rühren in die Lösung eingeleitet. Nach 3 h Rühren bei 0-3°C und wird die Mischung auf Raumtemperatur aufgewärmt. Nach vollständigem Entfernen des Lösungsmittels im Vakuum (10 mbar) werden 105 g (95 % der Theorie) 2-(Difluoracetyl)-3-(dimethylamino)-acrylsäureethylester in einer Reinheit von 98 F1.-% (GC Analyse) erhalten.
**¹H-NMR (CD₃CN):** δ= 7.88 (s, 1H), 6,47, 6,61 und 6.74 (t, 1H), 4.13-4.19 (m, 2H), 3.32 (s, 3H), 2.85 (s, 3H), 1.25-1.28 (t, 3H) ppm.

### Beispiel 2

71.6 g (0.5 mol) Dimethylaminoacrylsäureethylester werden in 150 ml Toluol gelöst, mit 0.5 mol Triethylamin versetzt und die Mischung auf 0°C gekühlt. Anschließend werden innerhalb von 30-40 min bei 0-3°C 50 g (0.5 mol) Difluoracetylfluorid unter Rühren in die Lösung eingeleitet. Nach 3 h Rühren bei 0-3°C wird die Reaktionsmischung auf Raumtemperatur aufgewärmt. Nach vollständigem Entfernen des Lösungsmittels im Vakuum (10 mbar) werden 108 g (98 % der Theorie) 2-(Difluoracetyl)-3-(dimethylamino)-acrylsäureethylester in einer Reinheit von 98 F1.-% (GC Analyse) erhalten.
**¹H-NMR (CD₃CN):** δ = 7.88 (s, 1H), 6,47, 6,61 und 6.74 (t, 1H), 4.13-4.19 (m, 2H), 3.32 (s, 3H), 2.85 (s, 3H), 1.25-1.28 (t, 3H) ppm.

### Beispiel 3

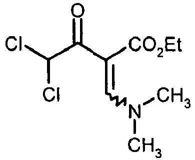

71.6 g (0.5 mol) Dimethylaminoacrylsäureethylester werden in 150 ml Toluol gelöst und unter Rühren bei 0-3°C zu einer Lösung von 73.7 g (0.5 mol) Dichloracetylfluorid getropft. Nach 3 h Rühren bei 0-3°C wird die Reaktionsmischung auf Raumtemperatur aufgewärmt. Nach vollständigem Entfernen des Lösungsmittels im Vakuum (10 mbar) erhält man 114 g (90 % der Theorie) des 2-(Dichloracetyl)-3-(dimethylamino)-acrylsäureethylesters. (Fp. 71-72°C).

### Beispiel 4

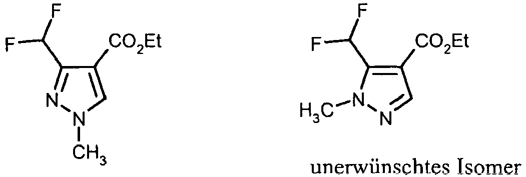

71.6 g (0.5 mol) Dimethylaminoacrylsäureethylester werden in 150 ml Toluol gelöst. Nach dem Abkühlen der Mischung auf 0°C, leitet man unter Rühren innerhalb von 30-40 min bei 0-3°C 50 g (0.5 mol) Difluoracetylfluorid in die Lösung ein. Im Anschluss daran wird die Mischung 3 h bei 0-3°C gerührt dann auf -20°C abgekühlt. Bei dieser Temperatur werden 26.4 g Methylhydrazin langsam zugetropft. Anschließend wird die Mischung weitere 3 h bei 0°C gerührt, auf Raumtemperatur aufgewärmt und abschließend 1 h bei 20-25°C gerührt.

Nach Zugabe von 500 ml Wasser wird die Toluolphase abgetrennt und die Wasserphase noch zweimal mit je 100 ml Toluol extrahiert. Nach dem Einengen der vereinigten Toluolphasen erhält man 3-(Difluormethyl)-1-methyl-1H-pyrazol-4-carbonsäureethylester (Ausbeute 89% der Theorie) im Gemisch mit dem unerwünschten Isomeren [5-(Difluormethyl)-1-methyl-1H-pyrazol-4-carbonsäureethylester] im Verhältnis von 91 : 9 (GC/MS-Analytik). Durch Waschen mit Hexan kann das unerwünschte Isomer vollständig entfernt werden. Ausbeute 85 %.

## Patentansprüche

1. Verfahren zum Herstellen von 2-Dihalogenacyl-3-amino-acrylsäureestern der Formel (I), die frei von Hydrochlorid Verunreinigungen sind, 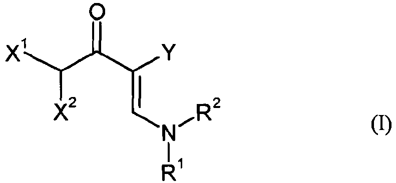 in welcher
R¹ und R² unabhängig voneinander ausgewählt sind aus C₁₋₁₂-Alkylresten, C₅₋₁₈-Aryl-, C₇₋₁₉-Alkylaryl- oder C₇₋₁₉-Arylalkyresten, oder
R¹ und R² zusammen mit dem Stickstoffatom, an das sie gebunden sind, einen 5- bis 6-gliedrigen Ring bilden können, der gegebenenfalls ein oder zwei weitere Hetero-Atome, ausgewählt aus O, S und einer SO₂-Gruppe enthalten kann,
Y ausgewählt ist aus (C=O)OR³, CN und (C=O)NR⁴R⁵, wobei R³, R⁴ und R⁵ unabhängig von einander ausgewählt sind aus C₁₋₁₂-Alkylresten, C₅₋₁₈-Aryl-, C₇₋₁₉-Alkylaryl- oder C₇₋₁₉-Arylalkyresten und R⁴ und R⁵ gemeinsam mit dem N-Atom, an das sie gebunden sind und/oder weiteren Atomen, die ausgewählt sind aus C, N, O und S einen fünf- oder sechsgliedrigen Ring bilden können, der mit C₁₋₆-Alkylresten substituiert sein kann; und
X¹ und X² unabhängig voneinander für Fluor, Chlor, Brom und Iod stehen,
durch Umsetzung von Säurefluoriden der Formel (II) 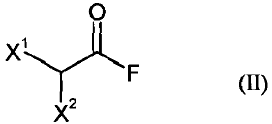 in welcher X¹ und X² die oben genannten Bedeutungen haben, mit 3-Amino-acrylsäure-Derivaten der Formel (III) 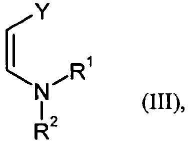 in welcher R¹, R² und Y die oben genannte Bedeutungen haben, **dadurch gekennzeichnet, dass** die Umsetzung in Abwesenheit von Basen erfolgt.

2. Verfahren gemäß Anspruch 1, wobei
R¹ und R² unabhängig voneinander ausgewählt sind aus Methyl, Ethyl, n-Propyl oder iso-Propyl, oder
R¹ und R² zusammen mit dem Stickstoffatom, an das sie gebunden sind, einen Piperidinyl- oder Pyrolidinyl-Ring bilden,
Y ausgewählt ist aus (C=O)OR³, wobei R³ ausgewählt ist aus Methyl, Ethyl, n-Propyl oder iso-Propyl.
X¹ und X² unabhängig voneinander ausgewählt sind aus Fluor, Chlor und Brom.

3. Verfahren gemäß Anspruch 1 oder 2, wobei
R¹ und R² Methyl sind,
Y (C=O)OC₂H₅ ist und
X¹ und X² beide Fluor sind.

4. Verfahren gemäß einem der Ansprüche 1 bis 3, wobei die Dialkylaminoacrylsäure-Derivate ausgewählt sind aus der Gruppe bestehend aus 3-(N,N-Dimethylamino)-acrylsäuremethylester, 3-(N,N-Dimethylamino)-acrylsäureethylester, 3-(N,N-Diethylamino)-acrylsäureethylester, 3-(N,N-Dimethylamino)-acrylsäurenitril, 3-(N,N-Dimethylamino)-acrylsäuredimethylamid und 3-(N,N-Dimethylamino)-acrylsäurediethylamid.

5. Verfahren gemäß einem der Ansprüche 1 bis 4, **dadurch gekennzeichnet, dass** die Umsetzung in einem organischen Lösungsmittel erfolgt.

## Claims

1. Process for preparing 2-dihaloacyl-3-aminoacrylic esters of the formula (I) that are free of hydrochloride impurities 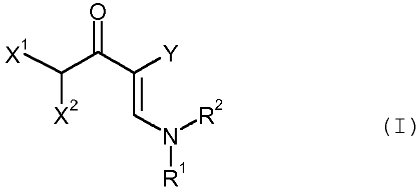 in which
R¹ and R² are each independently selected from C₁₋₁₂-alkyl radicals, C₅₋₁₈-aryl, C₇₋₁₉-alkylaryl and C₇₋₁₉-arylalkyl radicals, or
R¹ and R², together with the nitrogen atom to which they are bonded, may form a 5- to 6-membered ring which may optionally contain one or two further heteroatoms selected from 0, S and an SO₂ group,
Y is selected from (C=O)OR³, CN and (C=O)N⁴R⁵, where R³, R⁴ and R⁵ are each independently selected from C₁₋₁₂-alkyl radicals, C₅₋₁₈-aryl, C₇₋₁₉-alkylaryl and C₇₋₁₉-arylalkyl radicals, and R⁴ and R⁵, together with the nitrogen atom to which they are bonded and/or further atoms which are selected from C, N, 0 and S, may form a five- or six-membered ring which may be substituted by C₁₋₆-alkyl radicals; and
X¹ and X² are each independently fluorine, chlorine, bromine and iodine,
by reacting acid fluorides of the formula (II) 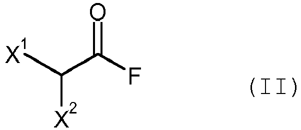 in which X¹ and X² are each as defined above with 3-aminoacrylic acid derivatives of the formula (III) 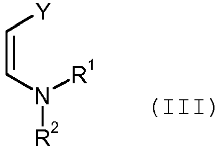 in which R¹, R² and Y are each as defined above, **characterized in that** the reaction is effected in the absence of bases.

2. Process according to Claim 1, wherein
R¹ and R² are each independently selected from methyl, ethyl, n-propyl and isopropyl, or
R¹ and R², together with the nitrogen atom to which they are bonded, form a piperidinyl or pyrrolidinyl ring,
Y is selected from (C=O)OR³ where R³ is selected from methyl, ethyl, n-propyl and isopropyl,
X¹ and X² are each independently selected from fluorine, chlorine and bromine.

3. Process according to Claim 1 or 2, wherein
R¹ and R² are each methyl,
Y is (C=O)OC₂H₅ and
X¹ and X² are both fluorine.

4. Process according to any one of Claims 1 to 3, wherein the dialkylaminoacrylic acid derivatives are selected from the group consisting of methyl 3-(N,N-dimethylamino)-acrylate, ethyl 3-(N,N-dimethylamino)acrylate, ethyl 3-(N,N-diethylamino)acrylate, 3-(N,N-dimethylamino)acrylonitrile, N,N-dimethyl-3-(N,N-dimethylamino)acrylamide and N,N-diethyl-3-(N,N-dimethylamino)acrylamide.

5. Process according to any one of Claims 1 to 4, **characterized in that** the reaction is effected in an organic solvent.

## Revendications

1. Procédé de fabrication d'esters de l'acide 2-dihalogénoacyl-3-amino-acrylique de formule (I), qui sont exempts d'impuretés chlorhydrate 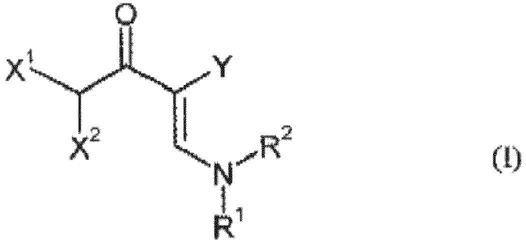 dans laquelle
R¹ et R² sont choisis indépendamment l'un de l'autre parmi les radicaux alkyle en C₁₋₁₂, les radicaux aryle en C₅₋₁₈, alkylaryle en C₇₋₁₉ ou arylalkyle en C₇₋₁₉, ou
R¹ et R² peuvent former ensemble avec l'atome d'azote auquel ils sont reliés un cycle de 5 à 6 éléments, qui peut éventuellement contenir un ou deux hétéroatomes supplémentaires choisis parmi 0, S et un groupe SO₂,
Y est choisi parmi (C=O)OR³, CN et (C=O)NR⁴R⁵, R³, R⁴ et R⁵ étant choisis indépendamment les uns des autres parmi les radicaux alkyle en C₁₋₁₂, les radicaux aryle en C₅₋₁₈, alkylaryle en C₇₋₁₉ ou arylalkyle en C₇₋₁₉, et R⁴ et R⁵ pouvant former ensemble avec l'atome N auquel ils sont reliés et/ou d'autres atomes choisis parmi C, N, 0 et S un cycle à cinq ou six éléments, qui peut être substitué avec des radicaux alkyle en C₁₋₆ ; et
X¹ et X² représentent indépendamment l'un de l'autre fluor, chlore, brome et iode,
par mise en réaction de fluorures d'acides de formule (II) 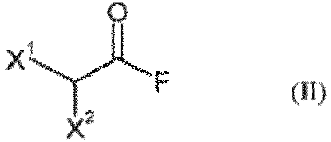 dans laquelle X¹ et X² ont les significations indiquées précédemment, avec des dérivés de l'acide 3-amino-acrylique de formule (III) 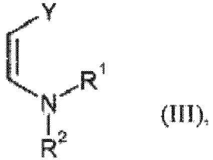 dans laquelle R¹, R² et Y ont les significations indiquées précédemment, **caractérisé en ce que** la réaction a lieu en l'absence de bases.

2. Procédé selon la revendication 1, dans lequel
R¹ et R² sont choisis indépendamment l'un de l'autre parmi méthyle, éthyle, n-propyle ou iso-propyle, ou
R¹ et R² forment ensemble avec l'atome d'azote auquel ils sont reliés un cycle pipéridinyle ou pyrrolidinyle, Y est choisi parmi (C=O)OR³, R³ étant choisi parmi méthyle, éthyle, n-propyle ou iso-propyle,
X¹ et X² sont choisis indépendamment l'un de l'autre parmi fluor, chlore et brome.

3. Procédé selon la revendication 1 ou 2, dans lequel
R¹ et R² représentent méthyle,
Y représente (C=O)OC₂H₅ et
X¹ et X² représentent tous les deux fluor.

4. Procédé selon l'une quelconque des revendications 1 à 3, dans lequel les dérivés de l'acide dialkylaminoacrylique sont choisis dans le groupe constitué par l'ester méthylique de l'acide 3-(N,N-diméthylamino)-acrylique, l'ester éthylique de l'acide 3-(N,N-diméthylamino)-acrylique, l'ester éthylique de l'acide 3-(N,N-diéthylamino)-acrylique, le nitrile de l'acide 3-(N,N-diméthylamino)-acrylique, le diméthylamide de l'acide 3-(N,N-diméthylamino)-acrylique et le diéthylamide de l'acide 3-(N,N-diméthylamino)-acrylique.

5. Procédé selon l'une quelconque des revendications 1 à 4, **caractérisé en ce que** la réaction a lieu dans un solvant organique.
